# EUROPÄISCHE PATENTSCHRIFT

(11) **EP 1 154 702 B1**
(45) Veröffentlichungstag und Bekanntmachung des Hinweises auf die Patenterteilung: **31.07.2002**
(21) Anmeldenummer: 00907609.2
(22) Anmeldetag: 23.02.2000
(51) Int. Cl.: A23J 3/34

(54) **VERFAHREN ZUR HERSTELLUNG EINES EIWEISSISOLATS AUS EINER EIWEISSHALTIGEN SUBSTANZ**
METHOD FOR PREPARING A PROTEIN ISOLATE FROM A SUBSTANCE CONTAINING PROTEINS
PROCEDE DE PREPARATION D'UN ISOLAT DE PROTEINES A PARTIR D'UNE SUBSTANCE CONTENANT DES PROTEINES

(30) Priorität: 23.02.1999 DE 19907725
(43) Veröffentlichungstag der Anmeldung: 21.11.2001
(73) Patentinhaber: NEUMÜLLER, Waldemar, D-37077 Göttingen (DE)
(72) Erfinder: NEUMÜLLER, Waldemar, D-37077 Göttingen (DE)
(74) Vertreter: Patentanwälte Rehberg + Hüppe
(86) Internationale Anmeldenummer: EP0001485
(87) Internationale Veröffentlichungsnummer: WO0049887

(56) Entgegenhaltungen:
- EP-A- 0 700 641
- WO-A-95/14394
- US-A- 4 443 540
- US-A- 4 559 307

## Beschreibung

Die Erfindung bezieht sich auf ein Verfahren zur Herstellung eines Eiweißisolats aus einer eiweißhaltigen Substanz nach dem Oberbegriff des Patentanspruchs 1.

Es sind mehrere Verfahren bekannt, um Eiweiß aus pflanzlichen Rohstoffen zu extrahieren, wobei Grundlage dieser Verfahren eine alkalische Extraktion des Eiweißes bei einem pH-Wert zwischen 7 und 9 und einer Temperatur von 40 bis 50°C ist. Das extrahierte Eiweiß wird anschließend durch Zentrifugation gereinigt und mit Mineralsäure solange angesäuert, bis der isoelektrische Punkt des Eiweißes erreicht ist. Das am isoelektrischen Punkt ausfallende Eiweiß wird erneut durch Zentrifugation konzentriert und gereinigt. Auf diese Weise sind Ausbeuten von 70 % bezogen auf das in den Rohstoffen enthaltene Eiweiß möglich. Allerdings ist von erheblichem Nachteil, daß nur Rohstoffe eingesetzt werden können, bei denen das Eiweiß eine geringe Denaturierung aufweist. Gerade für die Gewinnung des bedeutendsten pflanzlichen Eiweißisolats, des Soja-Eiweißisolats, stellt die Beschränkung auf die sog. "white flakes" als Rohstoff einen wirtschaftlichen Nachteil dar. White flakes fallen nur aufgrund eines speziellen Trocknungsverfahrens für die Reststoffe der Sojaölgewinnung an. Im Regelfall werden die Reststoffe weniger schonend getrocknet und liegen dann in Form sog. "toasted flakes" vor, in denen das Eiweiß stark denaturiert ist.

Aus der DE 1 203 588 ist es bekannt, den alkalischen Extraktionsprozeß bei der Gewinnung von Eiweißen aus einer eiweißhaltigen Substanz durch die vorherige Behandlung einer wässrigen Suspension der Substanz mit Wasserstoffperoxid im alkalischen Bereich und mit proteolytischen Enzymen zu fördern. Dazu wird der pH-Wert der Suspension der eiweißhaltigen Substanz zunächst angehoben, dann wird Wasserstoffperoxid zugegeben und die Temperatur erhöht, um eine Peroxidation auszulösen. Anschließend wird der pH-Wert auf 4-9 eingestellt, und es werden Enzyme zu der Suspension zugegeben, deren Aktivitätsminimum in diesem pH-Bereich liegt. Danach wird die Suspension zwei Stunden lang gerührt. Vorzugsweise finden pflanzliche Enzyme wie Bromelain, Ficin und Papain Verwendung. Nach der enzymatischen Hydrolyse wird das Eiweiß dadurch in Lösung gebracht, daß der pH-Wert auf 9-12 erhöht wird. Gleichzeitig kommen hohe Temperaturen zur Anwendung, von denen man inzwischen weiß, daß diese grade in Verbindung mit dem hohen pH-Wert zu Eiweißschäden führen. Der mehrfache Wechsel des pH-Werts bei diesem bekannten Verfahren macht eine großtechnische Anwendung aufwendig und führt zu einem hohen Verbrauch an Alkali und Säure, der mit einer starken Salzbildung einhergeht.

Aus der DE 44 29 787 C2 ist es ebenfalls bekannt, zur Verbesserung der Löslichheit von Eiweißen proteolytische Enzyme einzusetzen. Hier wird die Ausgangssubstanz zunächst mit Alkohol extrahiert, erst anschließend mechanisch zerkleinert und dann mittels Endo- und Exoproteasen im sauren bis neutralen Bereich weiter enzymatisch zerkleinert. Dabei erfolgt die Extraktion der Begleitstoffe des Eiweißes im Gegenstromverfahren. Auch bei diesem bekannten Verfahren kann nach der Extraktion des Eiweißes eine Behandlung mit Wasserstoffperoxid erfolgen. Das Verfahren ist durch den Einsatz von Alkohol zur Extraktion aufwendig und daher nachteilig. Es muß ständig die Explosionsgefährdung der Suspensionen berücksichtigt werden sowie die Inaktivierung der eingesetzten Enzyme durch vorhandenen Alkohol. Definitionsgemäß bleibt die Proteolyse bei dem bekannten Verfahren auf einen pH-Bereich von < 9,5 beschränkt, wobei in der DE 44 29 787 C2 nur der pH-Bereich von < 7,5 konkret dargestellt ist. Dem Fachmann ist aber umgekehrt bekannt, daß die Eiweißlöslichkeit dann besonders groß wird, wenn der pH-Wert über 9 liegt. Insgesamt ist der Wirkungsgrad des bekannten Verfahrens bezogen auf die aufzuwendenden Kosten für seine Umsetzung nur gering, so daß die erzeugten Eiweißisolate nicht wettbewerbsfähig sind.

Ein Verfahren nach dem Oberbegriff des Patentanspruchs 1 ist aus der DE 43 39 743 C1 bekannt. Dieses Verfahren kommt ohne die Verwendung von Alkohol aus. Zur Lösung der Proteine werden jedoch pH-Werte von über 11,5, insbesondere bei 12,5, benötigt, was mit einem hohen Verbrauch an Alkali und an Säure zur späteren Neutralisation verbunden ist. Vor der Proteolyse wird die eiweißhaltige Substanz mit einer Protease bei einem pH-Wert < 10,5 behandelt. Die Vorbehandlung erfordert dabei relativ große Einsatzmengen des Enzyms, was sich nachteilig auf die Produktionskosten auswirkt. Nachteilig ist auch der relativ hohe Salzgehalt am Ende der Eiweißextraktion bei diesem bekannten Verfahren, der in der Praxis zusätzliche Waschschritte erforderlich macht.

Aus der JP 20 76 597 A ist ebenfalls ein Verfahren zur Extraktion von Eiweißen aus einer eiweißhaltigen Substanz bekannt, das mit hohen Enzymmengen arbeitet. Dabei wird einer alkalischen Suspension der eiweißhaltigen Substanz, deren pH-Wert zwischen 10 und 13 liegt, eine alkalische Protease zugegeben und bei einer Temperatur von 30-50°C für 1-20 Stunden inkubiert. Anschließend erfolgt die Neutralisation und eine Filtration der Suspension. Diese recht drastischen Bedingungen der Eiweißextraktion führen zu umfangreichen Eiweißschäden. So ist es zwar möglich, durch das bekannte Verfahren Hydrolysate für kosmetische Verwendungen zu gewinnen, für die Herstellung von Eiweißisolaten als Nahrungsmittel ist es aber ungeeignet.

Als gemeinsamer Schritt der vorstehend im einzelnen beschriebenen Verfahren kann der Einsatz von eiweißspaltenden Enzymen vor der alkalischen Extraktion angesehen werden, der die Extrahierbarkeit der Eiweiße bei der anschließenden alkalischen Extraktion verbessert.

Aus der US-A-4,443,540 ist ein Verfahren zur Herstellung von Eiweißhydrolysaten bekannt, bei dem ein Eiweißmaterial mit mindestens einem proteolytischen Enzym bei kontrollierter Temperatur und alkalischem pH-Wert hydrolysiert wird. Das Eiweiß wird dann durch Filtration in ein Eiweißfiltrat und ein Eiweißretentat aufgespalten. Das Eiweißfiltrat enthält das hydrolysierte Eiweiß mit niedrigem Molekulargewicht. Das Eiweißretentat, welches neben Eiweiß mit höherem Molekulargewicht auch die proteolytischen Enzyme enthält, wird zumindest teilweise zurückgeführt, um die Eiweiße mit höherem Molekulargewicht einer weitergehenden Hydrolyse zu unterwerfen.

Die vorliegende Erfindung hat zur Aufgabe, ein Verfahren nach dem Oberbegriff des Patentanspruchs 1 aufzuzeigen, das sowohl bei eiweißhaltigen Substanzen mit hoher als auch mit geringer Eiweißlöslichkeit zu einer verbesserten Eiweißausbeute führt, bei gleichzeitig geringerem Einsatz der Hilfsmittel Wasser, Alkali, Säure und Enzym, ohne daß hierdurch die Qualität des erzeugten Eiweißisolats abfällt.

Erfindungsgemäß wird diese Aufgabe durch ein Verfahren mit dem Merkmalen des Patentanspruchs 1 gelöst.

Vorteilhafte Ausführungsformen dieses Verfahrens sind in den Unteransprüchen 2 bis 10 beschrieben.

Bei dem neuen Verfahren wird mit Hilfe einer alkalischen Gegenstromextraktion die Lösung des Eiweißes durch einen Temperaturgradienten, unterschiedlicher pH-Werte und gleichzeitiger Einwirkung von eiweißspaltenden Enzymen während der Extraktionskaskade durchgeführt. Im Einzelfall kann diese Extraktion durch den Einsatz von Wasserstoffperoxid und/oder Alkoholen unterstützt werden.

Zunächst wird die Ausgangssubstanz, wenn sie nicht bereits mit einer solchen kleiner Teilchengröße vorliegt, zu einem Mehl mit einer Teilchengröße von 50 bis 100 µm vermahlen und mit Wasser im Verhältnis 1:5 bis 1:8 vermischt, wobei die Suspension auf eine Temperatur von 30 bis 60°C, insbesondere von etwa 50°C, gebracht wird. Der pH-Wert der Suspension wird mittels Alkalioder Erdalkalihydroxiden auf 8 bis 10, insbesondere auf etwa 9, eingestellt. Um die Eiweißlöslichkeit in dieser ersten Extraktionsstufe zu steigern, kann hier bereits eine Protease zugesetzt werden. Vorzugsweise wird jedoch der geklärte Oberlauf bzw. das Filtrat der folgende Extraktionsstufe zugesetzt. Das dabei erreichte Enzym/Eiweiß-Verhältnis sollte zwischen 1:2000 und 1:6000 liegen. Nach einer Inkubationszeit von 10 bis 60 min., insbesondere von etwa 20 min., erfolgt eine erste Separation. Hierfür eignen sich grundsätzlich alle gängigen Trenntechniken. Bevorzugt ist die Verwendung eines Sedikanters der Firma Flottweg, mit dem ein Kraftfeld von 6000 x g für die Separation zur Verfügung steht. Bei der Separation kann eine Schaumbildung durch eine Vakuumumgebung weitgehend vermieden werden. Ein geeignetes Vakuum liegt im Bereich eines Absolutdrucks von 300 bis 500 mbar. So gelingt es, Eiweißsuspensionen ohne Entschäumer zu verarbeiten. Wenn es nicht möglich ist, die Separation unter Vakuum durchzuführen, ist für eine ausreichende Entgasung der Suspension vor der Separation zu sorgen, wenn keine Entschäumer eingesetzt werden sollen.

Nach der erfolgten Separation wird der nicht gelöste Feststoff mit Wasser dispergiert, anschließend homogenisiert und dann einer erneuten alkalischen Extraktion unterworfen. Hierzu wird die Temperatur auf 30 bis 45°C, insbesondere etwa 40°C, eingestellt, der pH-Wert auf 10,5 bis 11,5, insbesondere auf ca. 11,2. Grundsätzlich kann auf jeder Extraktionsstufe homogenisiert werden. Besonders effektiv ist jedoch die Homogenisation vor der hier beschriebenen Extraktionsstufe, die damit als ausreichend für die Durchführung des neuen Verfahrens anzusehen ist. Wichtig ist die Einbringung des geklärten Oberlaufs bzw. des Filtrats der nachfolgenden Extraktionsstufe in die hier beschriebene Extraktionsstufe, so daß ein Enzym/Eiweiß-Verhältnis von 1:500 bis 1.2000, insbesondere von etwa 1:1250, vorliegt. Nach einer Extraktionsdauer von 10 bis 60 min. erfolgt eine zweite Separation.

Anschließend wird der Feststoff zum dritten Mal mit Wasser suspendiert. Der pH-Wert stellt sich dabei je nach Wasserqualität auf 10,2 bis 10,5 ein. Die Temperatur wird auf 25 bis 40°C, insbesondere etwa 35°C, eingestellt. Dieser Suspension wird originär eine Protease zugesetzt. Das Enzym/Eiweiß-Verhältnis beträgt dabei 1:100 bis 1:500, insbesondere etwa 1:400. Nach Zugabe der Protease, bei der es sich auch um eine Proteasenmischung handeln kann, folgt eine enzymatische Hydrolyse für 3 bis 30 min.. Die Hydrolysedauer richtet sich nach der eingesetzten Enzymmenge, der jeweiligen Enzymaktivität und der Art der Protease. Geeignete Proteasen können aus den Gruppen der Serin-Proteasen, der Zystein-Proteasen, der Asparagin-Proteasen und/oder der Metall-Proteasen ausgewählt werden.

Verschiedene eiweißhaltige Substanzen als Rohstoffe für das neue Verfahren weisen eine Sensorik auf, die das entstehende Eiweißisolat ohne besondere Maßnahmen ungenießbar machen. Daher hat es sich als vorteilhaft bewiesen, während der zweiten alkalischen Extraktionsstufe Wasserstoffperoxid zuzusetzen. Dabei kann die Menge bezogen auf eine 35%ige Wasserstoffperoxidlösung zwischen 5 und 50 ml pro kg Eiweiß, insbesondere 20 ml/kg Eiweiß liegen. Die Konzentration ist so abzustimmen, daß eine Beschädigung der eingesetzten Proteasen nicht erfolgt.

Sollten in der eiweißhaltigen Ausgangssubstanz organische Stoffe enthalten sein, die sich nicht über eine wässrige Vorbehandlung entfernen lassen, ist es von Vorteil, die alkalische Extraktion in Anwesenheit von 5 bis 20 % Alkohol, insbesondere von 10 % Alkohol, durchzuführen. Hierdurch wird die Löslichkeit der organischen Stoffe erhöht, was dazu führt, daß diese bei der Fällung des Eiweißes am isoelektrischen Punkt überwiegend in der wässrigen Lösung verbleiben. Auch bei dem Alkohol ist es ausreichend, ihn in der zweiten Extraktionsstufe zuzugeben, wobei darauf zu achten ist, daß die gewünschte Alkoholkonzentration nach Rückführung des Klarlaufs der zweiten Separation in die erste Extraktionsstufe nicht unter 5 % liegen sollte.

Der Klarlauf der ersten Separationsstufe wird bei dem neuen Verfahren in an sich bekannter Weise mittels Mineralsäuren oder organischen Säuren auf den produktabhängigen isoelektrischen Punkt des Eiweißes, der gewöhnlich in dem pH-Bereich von 4,2-4,6 liegt, eingestellt. Dabei fällt das Eiweiß aus, und es kann eine Abtrennung mit bekannten Separationstechniken, insbesondere mit Dekantern, durchgeführt werden. Das so erhaltene quarkartige Eiweißisolat kann vor seiner Trocknung gewaschen und neutralisiert werden, um einen gewünschten pH-Wert für das Endprodukt einzustellen.

Überraschenderweise führt die stufenweise alkalische Extraktion unter Einsatz von Proteasen mit Rückführung der Extraktionshilfsmittel zu einer Eiweißqualität, die deutlich über derjenigen der Verfahrensprodukte von bekannten Verfahren liegt. Dies betrifft sowohl die Ausbeute als auch die Sensorik der Verfahrensprodukte. Gleichzeitig kann die Verfahrensführung bei dem neuen Verfahren so gewählt werden, daß mit einem Wasserverbrauch von ca. 8 l Frischwasser pro kg eingesetzter Rohstoffe über alle Verfahrensschritte auszukommen ist. Hiervon müssen nur ca. 25 % Warmwasser sein. Das anfallende Abwasser beträgt ca. 5 l je eingesetztem kg Rohstoff.

Durch die relativ großen Feststoffkonzentrationen der verarbeiteten Suspensionen werden bei dem neuen Verfahren trotz der höheren pH-Werte keine größeren Mengen Säure und/oder Alkali gegenüber klassischen Extraktionsverfahren verbraucht. Vielmehr ist gegenüber derzeitigen kommerziell eingesetzten Verfahren zur Gewinnung von Eiweißisolaten eine Reduktion der Säure- und Alkalimengen und damit der Versalzung um bis zu 20 % erreichbar.

Die durch das erfindungsgemäße Verfahren erhaltenen Eiweißisolate weisen einen typischen Eiweißgehalt von 90 bis 94 % bezogen auf den Trockensubstanzgehalt auf. Je nach Art der durchgeführten Trocknung kann der Restfeuchtegehalt bei 4 bei 8 % liegen. Die Ausbeuten bezogen auf den Eiweißgehalt der Ausgangssubstanz liegen typischerweise bei 80 bis 87 %. Der Geschmack der Verfahrensprodukte ist bei abgestimmter Verfahrensführung neutral. Ein etwaiger von der Ausgangssubstanz abhängiger Eigengeschmack kann ohne weiteres beseitigt werden. Die Proteinschäden sind sehr gering. Dies wird beispielsweise durch einen sehr geringen Lysinoalaningehalt von nur 50 bis 150 ppm bezogen auf das Eiweiß in dem erhaltenen Eiweißisolat dokumentiert.

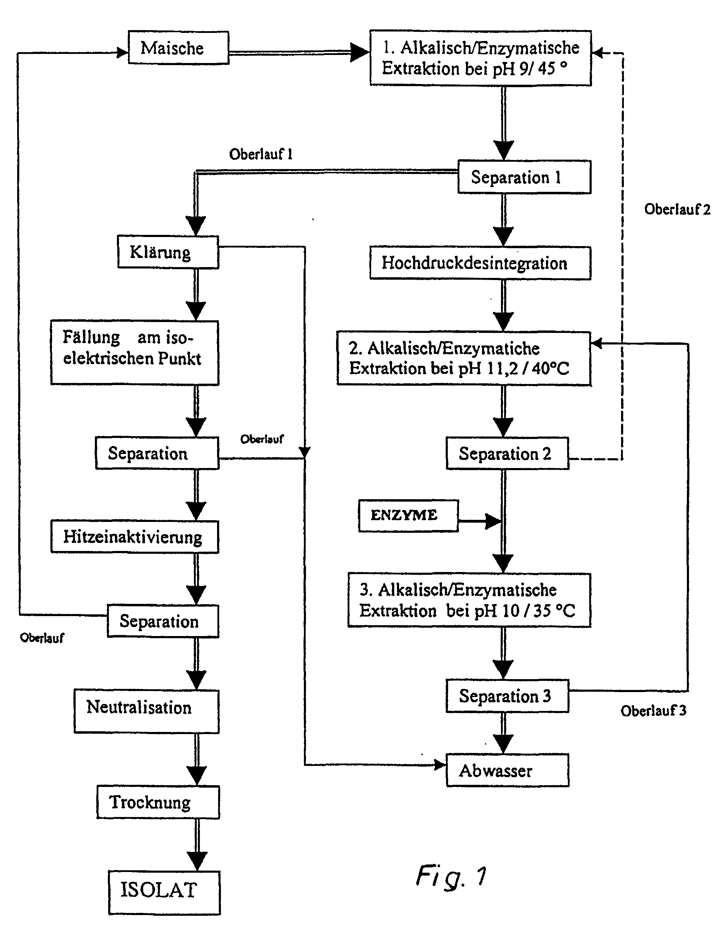

Das in der beigefügten Fig. 1 wiedergegebene Ablaufdiagramm des erfindungsgemäßen Verfahrens, bei dem die bevorzugten pH- und Temperaturbereiche angegeben sind, geht von einer Maische aus, die durch Vermischen der in kleinteiliger Form eingesetzten eiweißhaltigen Ausgangssubstanz mit dem Klarlauf einer Separationsstufe angesetzt wird. Im rechten Teil von Fig. 1 sind die drei alkalisch/enzymatischen Extraktionsstufen der bevorzugten Ausführungsform des neuen Verfahrens angedeutet. Im linken Teil ist die Aufarbeitung des Oberlaufs 1 der Separation 1, d.h. der Separation nach der ersten Extraktionsstufe, wiedergegeben. Dieser Oberlauf enthält das Eiweiß zunächst in gelöster Form. Nicht gelöste Substanzen in dem Oberlauf werden durch eine Klärung entfernt. Anschließend erfolgt eine Fällung der Eiweiße am isoelektrischen Punkt. Das Präzipitat dieser Fällung wird durch Separation abgetrennt und wieder mit Wasser suspendiert. Durch eine anschließende Hitzeinaktivierung werden die Enzyme aus der Extraktion des Eiweißes inaktiviert. Bei der folgenden Separation wird der Oberlauf gewonnen, mit dem die Maische angesetzt wird. Dieser Oberlauf enthält keine nennenswerte Enzymaktivität mehr. Die nachgeschaltete Neutralisation und Trocknung des Feststoffs ergibt das gewünschte Eiweißisolat.

Im folgenden wird die Erfindung anhand von Beispielen weiter erläutert und beschrieben:

### 1.) Soja-Eiweißisolat

Getoastetes Sojaschrot wird zunächst zu einem Eiweißkonzentrat aufgearbeitet. Das Eiweißkonzentrat, das eine Teilchengröße von ca. 50 µm aufweist, wird mit 50°C warmem Wasser aufgenommen, so daß eine Feststoffkonzentration der entstehenden Suspension von 12,5 % vorliegt. Dies entspricht der Maischen in Fig. 1. Dann wird diese Suspension mit dem Rücklauf aus der nachgeschalteten zweiten Extraktionsstufe zusammengeführt. Anschließend wird der pH-Wert mit 25%iger Natronlauge auf 9 eingestellt. Das Enzym/Eiweiß-Verhältnis beträgt 1:5500. Die Suspension wird 15 min. lang bei 45°C gerührt. Dann wird die Suspension über einen Sedikanter bei 6000 x g getrennt, wobei ein Vakuum von 300 mbar Absolutdruck im Sedikanter vorherrscht. Der Oberlauf des Sedikanters enthält das bereits gelöste Eiweiß, der Unterlauf den Feststoff mit dem noch gebundenen Eiweiß.

Der Unterlauf wird erneut mit Wasser aufgenommen, so daß ein Feststoffgehalt von 8 % eingestellt wird. Das Wasser kann kalt sein. Die so erhaltene Suspension wird mit einem Druckabfall von 80 bar homogenisiert und mit dem geklärten Oberlauf der dritten Extraktionsstufe zusammengeführt. Die sich einstellende Temperatur liegt bei 38°C, das Enzym/Eiweiß-Verhältnis bei 1:1250. Dann wird der pH-Wert mit 25%iger Natronlauge wieder auf 11,2 eingestellt, und es wird für 15 min. gerührt. Anschließend erfolgt eine zweite Separation über einen Sedikanter unter Vakuum. Der Oberlauf dieses Sedikanters wird in die erste Extraktionsstufe zurückgeführt. Der Feststoffaustrag, d.h. der Unterlauf, wird mit kaltem Wasser dispergiert und auf einen Feststoffgehalt von 6 % eingestellt. Die sich einstellende Temperatur sollte unter 40°C liegen. Zu dieser Suspension werden nunmehr Proteasen mit einer Gesamtaktivität von 6 Anson-Units/kg Protein zugegeben, was einem Enzym/Eiweiß-Verhältnis von 1:400 entspricht. Diese Suspension reagiert für 5 min., und wird dann in einer dritten Separation in einem Sedikanter unter Vakuum zentrifugiert. Der oberlauf wird der zweiten Extraktionsstufe zugeführt. Der Feststoffaustrag bzw. Unterlauf kann neutralisiert und beispielsweise als faserhaltiges Quellmittel verwendet werden. In dem hier beschriebenen Verfahren wird er nicht weiter verwendet.

Der Oberlauf der ersten Extraktionsstufe wird mit 15%iger Salzsäure auf einen pH-Wert von 4,3 eingestellt und mit einem Sedikanter bei 300 mbar zentrifugiert. Die Temperatur des Zulaufs zu dem Sedikanter liegt bei 45°C. Der Feststoffaustrag weist 28 % Trockensubstanz auf, d.h. der Unterlauf des Sedikanters enthält das Eiweiß, der Oberlauf die Begleitstoffe und lösliches Eiweiß, welches dem Abwasser zugeführt wird. Der Feststoff wird nunmehr mit Wasser bester Qualität auf 15 % verdünnt und kurzzeitig auf 110°C erhitzt. Hierdurch wird Feststoff gewaschen und die in ihm enthaltenen Enzyme werden inaktiviert. Nach Kühlung auf 50°C erfolgt eine Trennung des Feststoffs von dem zugesetzten Wasser auf einen normalen Dekanter bei ca. 4000 x g. Der Oberlauf dieser Separation wird zum Ansetzen der Maische für die erste Extraktionsstufe verwendet. Der Unterlauf wird mit Wasser erneut auf 15 % Trockensubstanzgehalt verdünnt, mit Natronlauge neutralisiert und anschließend sprühgetrocknet.

### 2.) Kartoffel-Eiweißisolat

Bei der Stärkegewinnung anfallende eiweißhaltige Beiprodukte werden zunächst auf eine Teilchengröße von 50 µm vermahlen und dann mit 50°C warmem Wasser so aufgenommen, daß eine Suspension mit einem Trockensubstanzgehalt von 15 % entsteht. Dieser Suspension wird eine Protease mit einer Aktivität von 5 Anson-Units/kg Eiweiß zugesetzt. Nach 5 min. Rührzeit wird der pH-Wert auf 8,5 eingestellt, und dann wird für weitere 10 min. gerührt. Anschließend wird der pH-Wert der Suspension mit Salzsäure auf 4,2 eingestellt, und die Suspension wird auf einem Dekanter bei 4000 x g zentrifugiert. Der Oberlauf wird verworfen, der Feststoff wird mit 50°C warmem Wasser erneut aufgenommen und dann gemäß den in Fig. 1 dargestellten Schritten des neuen Verfahrens aufbereitet. Hierzu wird dem im Wasser aufgenommenen Feststoff der Oberlauf der zweiten Extraktionsstufe zugesetzt. Das Enzym/Eiweiß-Verhältnis beträgt danach 1:6500. Die Feststoffkonzentration beträgt 10 %. Der pH-Wert wird auf 9 eingestellt, und anschließend wird für 15 min. gerührt. Dabei sollte die Temperatur 45°C betragen. Anschließend erfolgt eine Zentrifugation auf einem Sedikanter bei 6000 x g. Dabei enthält der Oberlauf das gelöste Protein. Der Unterlauf, d.h. der Feststoffaustrag mit einer Trockensubstanz von 26 %, wird mit kaltem Wasser aufgenommen und mit dem Oberlauf der nachfolgenden dritten Extraktionsstufe zusammengeführt. Die Temperatur stellt sich dabei auf 38°C ein. Der pH-Wert wird mit 25%iger Natronlauge auf 11,2 angehoben. Das Enzym/Eiweiß-Verhältnis beträgt 1:1600. Diese Suspension wird 15 min. lang gerührt, wobei nach 10 min. 20 µl 25%iges Wasserstoffperoxid/kg Feststoff zugesetzt werden. Nach der Rührzeit von 15 min. wird die Suspension über einen Druckabfall von 80 bar homogenisiert und dann über einen zweiten Sedikanter bei einem Unterdruck von 300 mbar aufgetrennt. Der Oberlauf wird der ersten Extraktionsstufe zugeführt; der Unterlauf mit dem Feststoff wird erneut mit kaltem Wasser aufgenommen. Die Temperatur des dispergierten Feststoffs stellt sich auf 32°C ein. Der pH-Wert erreicht 10,5. Hierzu werden nun Proteasen in einer Aktivität von 4 Anson-Units/kg Eiweiß zugesetzt, was zu einem Enzym/Eiweiß-Verhältnis von 1:500 führt. Nach 5 min. wird diese Dispersion ein drittes Mal auf einem Sedikanter wiederum unter einem Vakuum von 300 mbar Absolutdruck zentrifugiert. Der Oberlauf dieser Separation wird der zweiten Extraktionsstufe zugeführt. Der Feststoffaustrag, d.h. der Unterlauf wird verworfen.

Der Oberlauf der ersten Extraktionsstufe wird mit Salzsäure auf einen pH-Wert von 4,3 eingestellt. Anschließend wird mit einem Sedikanter bei einem Unterdruck von 300 mbar absolut zentrifugiert. Der Oberlauf aus dem Sedikanter wird verworfen. Der Unterlauf, der quarkartig ist, wird mit demineralisiertem Wasser auf 15 % Feststoffgehalt eingestellt. Diese Dispersion wird kurzzeitig auf 110°C erhitzt, dann auf 50°C abgekühlt und auf einem Dekanter bei 4000 x g zentrifugiert. Der dabei anfallende Klarlauf bzw. Oberlauf dient zum Anmaischen des Mehls der Ausgangssubstanz für die erste Extraktionsstufe. Der Feststoffaustrag, d.h. der Unterlauf wird neutralisiert und getrocknet.

Anstelle von Wasserstoffperoxid kann bei der Herstellung von Kartoffel-Eiweißisolat auch Ethanol zur Extraktion unerwünschter organischer Begleitstoffe eingesetzt werden.

## Patentansprüche

1. Verfahren zur Herstellung eines Eiweißisolats aus einer eiweißhaltigen Substanz, wobei die Substanz zu einem Mehl vermahlen wird, wobei das Mehl in einer wässrigen Lösung suspendiert wird, wobei das Eiweiß unter Einsatz mindestens einer im Gegenstrom geführten Protease aus dem Mehl in die Lösung gebracht wird, wobei das Eiweiß unter Einsatz einer Mineralsäure aus der Lösung ausgefällt und neutralisiert wird, **dadurch gekennzeichnet, daß** das Eiweiß durch eine mindestens zweistufige Behandlung mit der mindestens einen Protease, pH-Werten größer als 8 und Wärme von Temperaturen zwischen 30 und 60 °C aus dem Mehl in die Lösung gebracht wird, wobei das Mehl in der ersten Stufe bei einer niedrigeren Konzentration der mindestens einen Protease bezogen auf das Gewicht des Eiweißes, einem niedrigeren pH-Wert und einer höhere Temperatur als in der zweiten Stufe behandelt wird, wobei nach der ersten Stufe ein erster Oberlauf von einer das Mehl enthaltenden Fraktion separiert wird, aus dem das Eiweiß ausgefällt wird, und wobei nach der zweiten Stufe ein zweiter Oberlauf von einer das Mehl enthaltenden Fraktion separiert wird, der in die erste Stufe zurückgeführt wird.

2. Verfahren nach Anspruch 1, **dadurch gekennzeichnet, daß** die eiweißhaltige Substanz zu dem Mehl mit einer mittleren Teilchengröße von 30 bis 100 µm vermahlen wird.

3. Verfahren nach Anspruch 1 oder 2, **dadurch gekennzeichnet, daß** zu Beginn der zweiten Stufe das Mehl unter Energieeintrag in der Lösung homogenisiert wird.

4. Verfahren nach Anspruch 1, 2 oder 3, **dadurch gekennzeichnet, daß** das Eiweiß durch eine dreistufige Behandlung mit der mindestens einen Protease, pH-Werten größer als 8 und Wärme von Temperaturen zwischen 30 und 60 °C aus dem Mehl in die Lösung gebracht wird, wobei das Mehl in der dritten Stufe bei einer höheren Konzentration der mindestens einen Protease bezogen auf das Gewicht des Eiweißes und einer niedrigeren Temperatur als in der zweiten Stufe behandelt wird und wobei nach der dritten Stufe ein dritter Oberlauf von einer das Mehl enthaltenden Fraktion separiert wird, der in die zweite Stufe zurückgeführt wird.

5. Verfahren nach Anspruch 4, **dadurch gekennzeichnet, daß** die Protease der Lösung in der dritten Stufe zugegeben wird.

6. Verfahren nach einem der Ansprüche 1 bis 5, **dadurch gekennzeichnet, daß** die mindestens eine Protease eine Serin-, Cystein-, Asparagin- oder Metall-Protease ist.

7. Verfahren nach einem der Ansprüche 1 bis 6, **dadurch gekennzeichnet, daß** die mindestens eine Protease in dem Eiweiß durch Hitzebehandlung inaktiviert wird.

8. Verfahren nach einem der Ansprüche 1 bis 7, **dadurch gekennzeichnet, daß** das Eiweiß unter Einsatz mindestens eines Alkohols und/oder von Wasserstoffperoxid aus dem Mehl in die Lösung gebracht wird.

9. Verfahren nach einem der Ansprüche 1 bis 8, **dadurch gekennzeichnet, daß** das Mehl vor dem in die Lösung Bringen des Eiweißes einer Vorbehandlung unter Einsatz von Laugen, Säuren und/oder Enzymen unterworfen wird.

10. Verfahren nach einem der Ansprüche 1 bis 10, **dadurch gekennzeichnet, daß** mindestens einer der Oberläufe von der das Mehl enthaltenden Fraktion separiert wird, indem das in der Lösung suspendierte Mehl unter Vakuum zentrifugiert oder dekantiert wird.

## Claims

1. Method for preparing an albumin isolate from a substance containing albumin, the substance being ground to a flour, the flour being suspended in an aqueous solution, the albumin being extracted from the flour into the solution using at least one countercurrent protease, the albumin being precipitated from the solution using mineral acid and being neutralized, **characterized in that** the albumin is extracted from the flour into the solution by means of an at least two stage treatment with said at least one protease, with a pH greater than 8 and with heat of temperatures between 30 and 60 °C; the flour being treated in the first stage with a lower concentration of the at least one protease in relation to the albumin weight, with a lower pH and with a higher temperature than in the second stage; a first upper flow being separated from a fraction containing the flour, from which upper flow the albumin is precipitated; and a second upper flow being separated from a fraction containing the flour after the second stage, which upper flow is fed back to the fist stage.

2. Method according to claim 1, **characterized in that** the albumin containing substance is ground to a flour with an average particle size of 30 to 100 µm.

3. Method according to claim 1 or 2, **characterized in that** the flour is homogenized by energy input at the beginning of the second stage.

4. Method according to claim 1, 2 or 3, **characterized in that** the albumin is extracted from the flour into the solution by means of a three stage treatment with the at least one protease, with pH-values of greater than 8 and with heat of temperatures between 30 and 60 °C; the flour being treated in the third stage with a higher concentration of the at least one protease in relation to the albumin weight and with a lower temperature than in the second stage; and a third upper flow being separated from a fraction containing the flour after the third stage, which upper flow is fed back into the second stage.

5. Method according to claim 4, **characterized in that** the protease is added to the solution in the third stage.

6. Method according to any of the claims 1 to 5, **characterized in that** the at least one protease is a serine, cysteine, asparagines or metal protease.

7. Method according to any of the claims 1 to 6, **characterized in that** the at least one protease is inactivated in the albumin by means of a heat treatment.

8. Method according to any of the claims 1 to 7, **characterized in that** the albumin is extracted from the flour into the solution using at least one alcohol and/or hydrogen peroxide.

9. Method according to any of the claims 1 to 8, **characterized in that** the flour is subjected to a pre-treatment using lyes, acids and/or enzymes before extracting the albumin into the solution.

10. Method according to any of the claims 1 to 10, **characterized in that** at least one of the upper flows is separated from the fraction containing the flour **in that** the flour suspended in the solution is centrifugated on a decanter under vacuum.

## Revendications

1. Procédé de production d'un isolat de protéines à partir d'une substance contenant des protéines, dans lequel la substance est broyée en une farine, la farine est mise en suspension dans une solution aqueuse, les protéines sont extraites de la farine dans la solution par utilisation d'au moins une protéase introduite à contre-courant, les protéines sont précipitées de la solution et neutralisées par utilisation d'un acide minéral, **caractérisé en ce que** les protéines sont extraites de la farine dans la solution par un traitement en au moins deux étapes avec au moins une protéase, des pH supérieurs à 8 et chauffage à des températures comprises entre 30 et 60 °C, la farine est traitée au cours de la première étape avec une concentration par rapport au poids des protéines de la au moins une protéase plus basse, avec un pH plus faible et une température plus élevée qu'au cours de la deuxième étape, après la première étape une première couche supérieure séparée d'une fraction contenant la farine, les protéines sont précipitées de celle-ci, et après la deuxième étape une deuxième couche supérieure est séparée d'une fraction contenant la farine et recyclée dans la première étape.

2. Procédé selon la revendication 1, **caractérisé en ce que** la substance contenant les protéines est broyée en farine dont la taille moyenne des particules est comprise entre 30 et 100 µm.

3. Procédé selon la revendication 1 ou 2, **caractérisé en ce qu'**au début de la deuxième étape la farine est homogénéisée par apport d'énergie dans la solution.

4. Procédé selon la revendication 1, 2 ou 3, **caractérisé en ce que** les protéines sont extraites de la farine dans la solution par un traitement en trois étapes avec au moins une protéase, des valeurs de pH supérieures à 8 et chauffage à des températures comprises entre 30 et 60 °C, la farine est traitée au cours de la troisième étape à une concentration de la au moins une protéase par rapport au poids des protéines plus élevé et à une température plus basse qu'au cours de la deuxième étape, et après la troisième étape, une troisième couche supérieure est séparée d'une fraction contenant la farine et recyclée dans la deuxième étape.

5. Procédé selon la revendication 4, **caractérisé en ce que** la protéase est ajoutée à la solution au cours de la troisième étape.

6. Procédé selon l'une des revendications 1 à 5, **caractérisé en ce que** la au moins une protéase est une sérine-protéase, une cystéine-protéase, une asparagine-protéase ou une métal-protéase.

7. Procédé selon l'une des revendications 1 à 6, **caractérisé en ce que** la au moins une protéase est désactivée dans les protéines par traitement par la chaleur.

8. Procédé selon l'une des revendications 1 à 7, **caractérisé en ce que** les protéines sont extraites de la farine dans la solution par utilisation d'au moins un alcool et/ou de peroxyde d'hydrogène.

9. Procédé selon l'une des revendications 1 à 8, **caractérisé en ce qu'**avant l'extraction des protéines dans la solution, la farine est soumise à un pré-traitement par utilisation de bases, d'acides et/ou d'enzymes.

10. Procédé selon l'une des revendications 1 à 9, **caractérisé en ce qu'**au moins l'une des couches supérieures est séparée de la fraction contenant la farine, par centrifugation sous vide ou décantation de la farine en suspension dans la solution.
